(19) Europäisches Patentamt
European Patent Office
Office européen des brevets

(11) Veröffentlichungsnummer: **0 410 389 B1**

# (12) EUROPÄISCHE PATENTSCHRIFT

(45) Veröffentlichungstag der Patentschrift: **15.03.95**

(51) Int. Cl.$^6$: **C07D 498/22**, A61K 31/55

(21) Anmeldenummer: **90114182.0**

(22) Anmeldetag: **24.07.90**

Die Akte enthält technische Angaben, die nach dem Eingang der Anmeldung eingereicht wurden und die nicht in dieser Patentschrift enthalten sind.

## (54) Indolocarbazol und dessen Verwendung.

(30) Priorität: **25.07.89 DE 3924538**

(43) Veröffentlichungstag der Anmeldung:
**30.01.91 Patentblatt 91/05**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 296 110**
**EP-A- 0 303 697**

**HETEROCYCLES, vol. 21, no. 1, 1984 WEINREB et al. "Natural pro- duct synthesisvia cyclo- additions with N-sulfinyl dienophiles" Seiten 309-324**

**THE JOURNAL OF ORGANIC CHEMISTRY, vol. 54, no. 4, 1989 J. BERGMAN et al."Synthesis of Indolo(2,3-a)pyrrolo(3,4-c)carbazoles by Double Fischer Indolizations" p. 824-828**

(73) Patentinhaber: **GÖDECKE AKTIENGESELLSCHAFT**
**Salzufer 16**
**D-10587 Berlin (DE)**

(72) Erfinder: **Kleinschroth, Jürgen, Dr.**
**Ricarda-Huch-Strasse 11**
**D-7809 Denzlingen (DE)**
Erfinder: **Schächtele, Christoph, Dr.**
**Darriwald 16**
**D-7800 Freiburg (DE)**
Erfinder: **Hartenstein, Johannes, Dr.**
**Fohrenbühl 23**
**D-7801 Stegen-Wittental (DE)**
Erfinder: **Rudolph, Claus, Dr.**
**Riedmattenstrasse 11**
**D-7801 Vörstetten (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft das Indolocarbazol 9,10,11,12-Tetrahydro-9,12-epoxy-1H-diindolo[1,2,3-fg:3',2',1'-kl]pyrrolo-[3,4-i][1,6]benzodiazocin-1,3(2H)-dion (I)

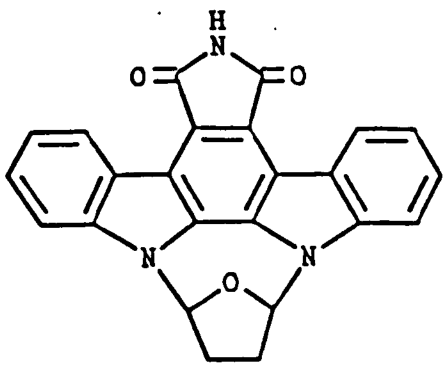

(I)

sowie Arzneimittel mit einem Gehalt an dieser Verbindung und das bei der Herstellung von (I) anfallende am Imidstickstoffatom durch einen 5-Methoxytetrahydrofuran-2-ylrest substituierte Derivat 9,10,11,12-Tetrahydro-2-(tetrahydro-5-methoxy-2-furanyl)-9,12-epoxy-1H-diindolo[1,2,3-fg:3',2',1'-kl]-pyrrolo-[3,4-i][1,6]-benzodiazocin-1,3(2H)-dion (IV), sowie deren Verwendung zur Herstellung von Arzneimitteln zur Behandlung von Herz- und Gefäßkrankheiten wie Thrombosen, Arteriosklerose, Hypertension und Entzündungsprozessen, Allergien, Krebs und bestimmten degenerativen Schäden des Zentralnervensystems sowie von Krankheiten des Immunsystems.

N,N-Glycoside von Indolocarbazolen als Inhibitoren von Serin/Threonin-Proteinkinasen sind bereits in der Literatur beschrieben worden. Als Referenzverbindung dieser Klasse von Proteinkinaseinhibitoren ist Staurosporin, ein Alkaloidglykosid mikrobiellen Ursprungs, anzusehen (Biochemical and Biophysical Research Communication 1986, 135, 397). Ein Nachteil dieses sehr potenten Inhibitors ist, daß er die verschiedenen Serin/Threonin-spezifischen Proteinkinasen wie Proteinkinase C, die cAMP- und die cGMP-abhängige Proteinkinase in gleichem Maße hemmt.

Aus EP-A-296110 sind am Methylamino-Stickstoff substituierte Derivate des Staurosporins bekannt, die Hemmwirkung auf die Proteinkinase C aufweisen.

Überraschenderveise wurde nun gefunden, daß die erfindungsgemäße Verbindung im Gegensatz zu Staurosporin eine hohe Selektivität für die Proteinkinase C aufweist. Es ist zwar in der Literatur schon die analoge Verbindung 2-Benzyl-9,12-epoxy-1-oxo-1H-2,3,9,10,11,12-hexahydro-diindolo[1,2,3-fg:3',2',1'-k1]-pyrrolo-[3,4-i][1,6]benzodiazocin (III)

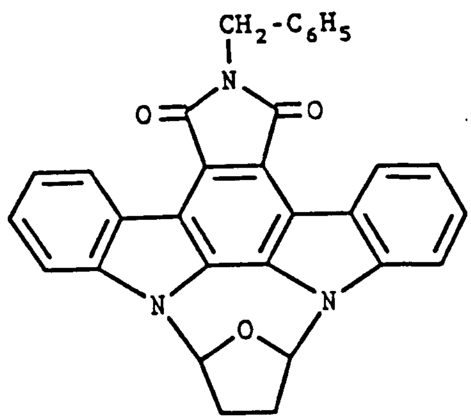

(III)

beschrieben worden, die sich von der erfindungsgemäßen Verbindung durch einen Benzylsubstituenten am Imid-Stickstoff unterscheidet (Heterocycles, 1984, 21, 309).

In der Literatur finden sich jedoch keine Angaben über eine inhibitorische Wirksamkeit gegenüber Proteinkinasen. Vergleichsversuche (Tabelle 1) zeigen jedoch, daß unter denselben Bedingungen des in-vitro-Enzym-Assays die Verbindung III bei einer Konzentration von $10^{-5}$ M keine signifikante Hemmung der Proteinkinase C besitzt. Die außergewöhnliche Potenzsteigerung beim Übergang von der N-Benzylverbindung III zur am Imid-Stickstoff unsubstituierten Verbindung I war überraschend.

Die Herstellung der erfindungsgemäßen Verbindung erfolgt in Analogie zu einem in der Literatur beschriebenem Verfahren (Heterocycles, 1984, 21, 309) durch Umsetzung von Indolo[2,3-a]pyrrolo[3,4-c]-carbazol-5,7(6H)-dion (II)

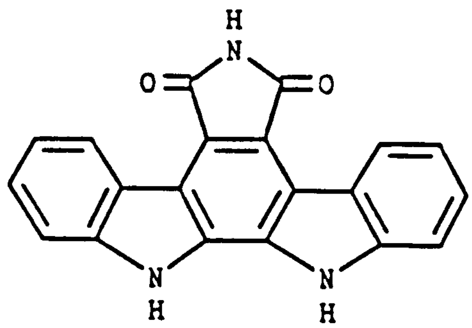

(II)

mit 2,5-Dimethoxytetrahydrofuran in Gegenwart eines sauren Katalysators, wobei vorzugsweise halogenierte Kohlenwasserstoffe, wie Dichlormethan oder 1,2-Dichlorethan als Lösungsmittel dienen. Als Katalysatoren sind vor allem p-Toluolsulfonsäure und Eisen(III)chlorid Hexahydrat geeignet.

Die Synthese des als Ausgangsprodukt dienenden Indolocarbazols (II) ist in der Literatur beschrieben (Tetrahedron Lett. 1983, 1441; J. Org. Chem. 1987, 52, 1177; J. Org. Chem. 1989, 54, 824).

Die Hemmung der mit Phosphatidylserin und Diacylglycerol aktivierten Proteinkinase C wurde nach EP-A-02 55 126 bestimmt. Die Bestimmung der Inhibierung der cAMP- und der cGMP-abhängigen Proteinkinase erfolgte gemäß den Testbeschreibungen im experimentellen Teil.

Tabelle 1

| | Inhibierung ($IC_{50}$; µM) | | | Verhältnis | $\frac{IC_{50}\text{Kinase}}{IC_{50}\text{ C-Kinase}}$ |
|---|---|---|---|---|---|
| Verbindung | C-Kinase | A-Kinase | G-Kinase | A / C | G / C |
| I | 0.028 | 5.5 | 0.52 | 196 | 19 |
| Staurosporin | 0.013 | 0.04 | 0.018 | 3.1 | 1.4 |
| III | > 10 | | | | |

Die Proteinkinase C spielt für die intracelluläre Signaltransduktion eine wichtige Schlüsselrolle und ist eng mit der Regulation von kontraktilen, sekretorischen und proliferativen Prozessen verknüpft. Aufgrund dieser Eigenschaften kann die erfindungsgemäße Verbindung zur Behandlung von Herz- und Gefäßkrankheiten wie Thrombosen, Arteriosklerose, Hypertension und Entzündungsprozessen, Allergien, Krebs und bestimmten degenerativen Schäden des Zentralnervensystems sowie von Krankheiten des Immunsystems verwendet werden. Die Verbindung kann in der jeweils geeigneten Formulierung enteral oder parenteral in Dosen von 1 bis 100 mg/kg, bevorzugt 1 bis 50 mg/kg verabreicht werden.

Der Gegenstand der Erfindung wird durch die folgenden Beispiele näher erläutert:
9,10,11,12-Tetrahydro-9,12-epoxy-1H-diindolo[1,2,3-fg:3′,2′,1′-kl]-pyrrolo-[3,4-i][1,6]benzodiazocin-1,3(2H)-dion (I):
Eine Suspension von 500 mg (1,54 mmol) Indolo[2,3-a]pyrrolo[3,4-c]carbazol-5,7-(6H)-dion (II) in 25 ml Dichlormethan wird mit 3 ml (23 mmol) 2,5-Dimethoxytetrahydrofuran, sowie 20 mg p-Toluolsulfonsäure versetzt und 5 Tage bei 20 °C gerührt. Das Lösungsmittel wird abdestilliert und der Rückstand zwischen Ethylacetat (400 ml) und Wasser (100 ml) verteilt. Die Ethylacetatphase wird abgetrennt, getrocknet (Natriumsulfat) und eingedampft. Der Rückstand wird an Kieselgel mit Toluol/Ethylacetat 3:1 chromatographiert. Die Fraktion mit dem Rf 0,45 wird isoliert und mit Diisopropylether/Ethylacetat verrührt.

Man erhält 9,10,11,12-Tetrahydro-9,12-epoxy-1H-diindolo[1,2,3-fg:3′,2′,1′-kl]-pyrrolo-[3,4-i][1,6]-benzodiazocin-1,3(2H)-dion (I) in Form gelber Kristalle, die >340 °C schmelzen.

Als Nebenprodukt wird bei der Chromatographie das am Imidstickstoffatom durch einen 5-Methoxytetrahydrofuran-2-ylrest substituiertes Derivat 9,10,11,12-Tetrahydro-2-(tetrahydro-5-methoxy-2-furanyl)-9,12-epoxy-1H-diindolo[1,2,3-fg:3′,2′,1′-kl]-pyrrolo-[3,4-i][1,6]benzodiazocin-1,3(2H)-dion (IV) erhalten (Rf 0,5 in Toluol/Ethylacetat 3:1).

Spektroskopische Daten von 9,10,11,12-Tetrahydro-2-(tetrahydro-5-methoxy-2-furanyl)-9,12-epoxy-1H-diindolo[1,2,3-fg:3′,2′,1′-kl]-pyrrolo-[3,4-i][1,6]benzodiazocin-1,3(2H)-dion(IV):
MS (70eV): m/z = 393 ($M^+$, 100), 321(26);
IR (KBr): = 3430, 1760, 1695, 1350, 1310, 740 $cm^{-1}$;
$^1$H-NMR (DMSO-$d_6$/TMS): $\delta$ = 2.04 (ps-q,2H,$CH_2$); 2.74 (m,2H, $CH_2$); 7.33 (m,2H,CH); 7.43 (t,2H,J = 7Hz,8Hz,ArH); 7.63 (t,2H,J = 7Hz,8Hz,ArH); 7.94 (d,2H,J = 8Hz,ArH); 9.01 (d,2H,J = 8Hz,ArH).

Testbeschreibung für G-Kinase und A-Kinase

cGMP-abhängige Proteinkinase: Das Enzym wird aus Rinderlunge gereinigt und seine Aktivität über den Einbau von Phosphor-32-markiertem Phosphat in Histon bestimmt. Im Testansatz von 200 µl sind folgende Komponenten enthalten: 20 mM Tris-HCl, pH 7.4, 5 mM $MgCl_2$, 1 mM DTT, 10 µM ATP, 10 µM cGMP, 40 µg BSA, 2% Glycerin sowie 10 µg Histon II-A und gegebenenfalls die zu untersuchende Testsubstanz. Der Ansatz wird ohne Enzym für 4 min bei 30 °C vorinkubiert und die Reaktion dann durch Zugabe von 2.5 nM G-Kinase gestartet. Nach 5 min Inkubation bei 30 °C wird die Reaktion durch Zugabe von 10% Trichloressigsäure gestoppt und die Proben dann über ein Nitrocellulosefilter abgefiltert. Der Phosphat-Einbau wird über Cerenkov-Zählung im Scintillationszähler bestimmt und daraus die prozentuale Inhibierung berechnet.

cAMP-abhängige Proteinkinase: Die Messung der Aktivität erfolgt mit der kommerziell verfugbaren katalytischen Untereinheit des Enzyms. Dabei wird der Einbau von Phosphor-32-markiertem Phosphat in Histon gemessen. Der Reaktionsansatz von 200 µl enthält folgende Komponenten: 50 mM PIPES-NaOH, pH 7.5, 10 mM $MgCl_2$, 1 mM DTT, 40 µM ATP sowie 50 µg Histon-H-2B. Die Durchführung des Tests erfolgt wie bei der G-Kinase. Der Start der Reaktion erfolgt dabei durch Zugabe von 6 Units der katalytischen Untereinheit der cAMP-abhängigen Proteinkinase.

## Patentansprüche

1. 9,10,11,12-Tetrahydro-9,12-epoxy-1H-diindolo[1,2,3-fg:3',2',1'-kl]-pyrrolo-[3,4-i][1,6]benzodiazocin-1,3-(2H)-dion.

2. Verfahren zur Herstellung der Verbindung gemäß Anspruch 1 dadurch gekennzeichnet, daß Indolo[2,3-a]pyrrolo[3,4-c]carbazol-5,7-(6H)-dion mit 2,5-Dimethoxytetrahydrofuran in Gegenwart eines sauren Katalysators und in einem halogenierten Kohlenwasserstoff als Lösungsmittel zu 9,10,11,12-Tetrahydro-9,12-epoxy-1H-diindolo[1,2,3-fg:3',2',1'-kl]-pyrrolo-[3,4-i][1,6]benzodiazocin-1,3(2H)-dion und dem Nebenprodukt 9,10,11,12-Tetrahydro-2-(tetrahydro-5-methoxy-2-furanyl)-9,12-epoxy-1H-diindolo[1,2,3-fg:3',2',1'-kl]-pyrrolo-[3,4-i][1,6 ]benzodiazocin-1,3(2H)-dion umgesetzt werden.

3. 9,10,11,12-Tetrahydro-2-(tetrahydro-5-methoxy-2-furanyl)-9,12-epoxy-1H-diindolo[1,2,3-fg:3',2',1'-kl]-pyrrolo-[3,4-i][1,6]benzodiazocin-1,3(2H)-dion.

4. Arzneimittel enthaltend neben üblichen Hilfs- und Zusatzstoffen mindestens eine der Verbindungen nach Anspruch 1 oder 3.

5. Verwendung der Verbindungen gemäß Anspruch 1 oder 3 zur Herstellung von Arzneimitteln zur Behandlung von Herz- und Gefäßkrankheiten wie Thrombosen, Arteriosklerose, Hypertension und Entzündungsprozessen, Allergien, Krebs und bestimmten degenerativen Schäden des Zentralnervensystems sowie von Krankheiten des Immunsystems.

## Claims

1. 9,10,11,12-Tetrahydro-9,12-epoxy-1H-diindolo[1,2,3- fg:3',2',1'-kl]pyrrolo[3,4-i][1,6]benzodiazocine-1,3-(2H)-dione.

2. A process for the preparation of the compound according to Claim 1, characterized in that indolo[2,3-a]-pyrrolo[3,4-c]carbazole-5,7(6H)-dione is reacted with 2,5-dimethoxytetrahydrofuran, in the presence of an acid catalyst and in a halogenated hydrocarbon as a solvent, to give 9,10,11,12-tetrahydro-9,12-epoxy-1H-diindolo[1,2,3-fg: 3',2',1'-kl]pyrrolo[3,4-i][1,6]benzodiazocine-1,3(2H)-dione and the by-product 9,10,11,12-tetrahydro-2-(5-methoxytetrahydrofuran-2-yl)-9,12-epoxy-1H-diindolo[1,2,3-fg:3',2',1'-kl]-pyrrolo[3,4-i][1,6]benzodiazocine-1,3(2H)-dione.

3. 9,10,11,12-Tetrahydro-2-(5-methoxytetrahydrofuran-2- yl)-9,12-epoxy-1H-diindolo[1,2,3-fg:3',2',1'-kl]-pyrrolo[3,4-i][1,6]benzodiazocine-1,3(2H)-dione.

4. A medicament containing at least one compound according to Claim 1 or 3, together with conventional adjuncts and additives.

5. Use of the compounds according to Claim 1 or 3 for the preparation of medicaments for the treatment of heart and vascular diseases such as thrombosis, arteriosclerosis, hypertension and inflammatory processes, allergies, cancer and certain degenerative impairments of the central nervous system, and diseases of the immune system.

## Revendications

1. 9,10,11,12-tetrahydro-9,12-époxy-1H-diindolo[1,2,3-fg:3',2',1'-kl]-pyrrolo-[3,4-i][1,6]benzodiazocin-1,3-(2H)-dione.

2. Procédé d'obtention d'un composé selon la revendication 1, caractérisé en ce que l'on fait réagir l'indolo[2,3-a]pyrrolo[3,4-c]-carbazol-5,7-(6H)-dione avec le 2,5-diméthoxytetrahydrofuranne en présence d'un catalyseur acide et dans un hydrocarbure halogéné utilisé en tant que solvant, pour le transformer en 9,10,11,12-tetrahydro-9,12-époxy-1H-diindclo[1,2,3-fg:3',2',1'-kl]pyrrolo-[3,4-i][1,6]-benzodiazocin-1,3(2H)-dione et en le sous produit consistant en 9,10,11,12-tetrahydro-2-(tetrahydro-5-méthoxy-2-furanyl)-9,12-époxy-1H-diindolo[1,2,3-fg:3',2',1'-kl]-pyrrolo-[3,4-i][1,6]benzodiazocin-1,3(2H)-dione.

3. 9,10,11,12-tetrahydro-2-(tetrahydro-5-méthoxy-2-furanyl)-9,12-époxy-1H-diindolo[1,2,3-fg:3',2',1'-kl]-pyrrolo-[3,4-i][1,6]benzodiazocin-1,3(2H)-dione.

4. Médicaments contenant, en plus des additifs et adjuvants classiques, au moins un composé selon la revendication 1 ou la revendication 3.

5. Application des composés selon la revendication 1 ou la revendication 3 à la fabrication de médicaments pour le traitement des maladies cardiaques et vasculaires telles que thrombose, artérosclérose, hypertension et processus inflammatoires, allergie, cancer et certains troubles dégénératifs du système nerveux central ainsi que des maladies du système immunitaire.